# EUROPÄISCHE PATENTSCHRIFT

(11) **EP 1 796 561 B1**
(45) Veröffentlichungstag und Bekanntmachung des Hinweises auf die Patenterteilung: **03.05.2017**
(21) Anmeldenummer: 05796158.3
(22) Anmeldetag: 06.10.2005
(51) Int. Cl.: A61B 17/80, A61B 17/86

(54) **KNOCHENSCHRAUBE**
BONE SCREW
VIS A OS

(30) Priorität: 08.10.2004 DE 102004050040
(43) Veröffentlichungstag der Anmeldung: 20.06.2007
(73) Patentinhaber: Aesculap AG, 78532 Tuttlingen (DE)
(72) Erfinder: PEUKERT, Andrea, 78532 Tuttlingen (DE); BEGER, Jens, 78532 Tuttlingen (DE); HAAS, Alexander, 78166 Donaueschingen (DE)
(74) Vertreter: Hoeger, Stellrecht & Partner Patentanwälte mbB
(86) Internationale Anmeldenummer: PCT/EP2005/010759
(87) Internationale Veröffentlichungsnummer: WO 2006/040063

(56) Entgegenhaltungen:
- EP-A- 0 809 974
- EP-A- 1 306 058
- US-A- 5 902 303
- US-A- 6 039 740
- US-A- 6 117 173
- US-A1- 2004 127 904

## Beschreibung

Die vorliegende Erfindung betrifft eine Knochenschraube mit einem eine Längsachse definierenden Schaft und mit einem Kopf, welcher mit einer Knochenschraubenaufnahme einer Knochenplatte in Eingriff bringbar ist zum lösbaren Verbinden der Knochenschraube mit der Knochenplatte, wobei ein Sicherungselement zum Sichern einer Verbindung der Knochenschraube und der Knochenplatte vorgesehen ist, wobei die Knochenschraube von einer Eingriffsstellung, in welcher die Knochenschraube an der Knochenplatte gehalten ist, in eine Lösestellung bringbar ist, in welcher die Knochenschraube von der Knochenplatte lösbar ist, wobei das Sicherungselement von einer Entsicherungsstellung, in welcher die Knochenschraube in die Lösestellung bringbar ist, in eine Sicherungsstellung zum Sichern der Verbindung zwischen der Knochenschraube und der Knochenplatte bringbar ist, in welcher die Knochenschraube die Eingriffsstellung einnimmt, wobei die Knochenschraube in einer Grundstellung, in welcher keine äußeren Kräfte auf sie wirken, die Eingriffsstellung einnimmt, wobei der Schaft eine Sicherungselementaufnahme aufweist und das Sicherungselement in der Sicherungselementaufnahme gelagert ist, wobei nach dem Einführen des Sicherungselements in die Sicherungselementaufnahme das Sicherungselement in axialer Richtung zwischen einem in distaler und einem in proximaler Richtung wirkenden Anschlag bewegbar ist und wobei das Sicherungselement in der Grundstellung der Knochenschraube in der Sicherungsstellung gehalten ist.

Knochenschrauben der eingangs beschriebenen Art sind beispielsweise aus der EP 1 306 058 A2, der US 5,902,303, der US 6,117,173, der EP 0 809 974 A2 sowie der US 6,039,740 bekannt und werden in der Chirurgie verwendet, um Knochenplatten an Knochenteilen eines menschlichen oder tierischen Körpers festzulegen. Vorteil dieser Schrauben ist es, daß die Eingriffsstellung verriegelbar ist, so daß sich die Knochenplatte unter Belastung nicht von der oder den Knochenschrauben lösen kann. Bekannt sind beispielsweise Verriegelungselemente, die an der Knochenplatte angeordnet sind und über den Kopf der eingedrehten Knochenschraube geschoben werden. Dies hat den Nachteil, daß an jeder Durchbrechung der Knochenplatte ein Sicherungselement vorgesehen werden muß. Werden jedoch nicht alle Knochenschraubenaufnahmen der Knochenplatte benötigt, so sind auch die an den nicht verwendeten Knochenschraubenaufnahmen angeordneten Sicherungselemente im Grunde genommen überflüssig. Des weiteren muß vor dem Einsetzen der Knochenplatte sichergestellt sein, daß alle Sicherungselemente die Entsicherungsstellung einnehmen. Ist dies nicht der Fall, dann müssen alle Sicherungselemente während des Eingriffs von der Sicherungsstellung in die Entsicherungsstellung überführt werden, was eine Operationszeit unnötig verlängert.

Es ist daher Aufgabe der vorliegenden Erfindung, eine Knochenschraube der eingangs beschriebenen Art so zu verbessern, daß eine Knochenplatte an Knochenteilen einfacher und sicherer festgelegt werden kann.

Diese Aufgabe wird bei einer Knochenschraube der eingangs beschriebenen Art erfindungsgemäß dadurch gelöst, daß das Sicherungselement in der Sicherungsstellung unter Vorspannung gehalten ist.

Durch die bewegliche Lagerung des Sicherungselements an der Knochenschraube werden nur so viele Sicherungselemente benötigt, wie tatsächlich Knochenschrauben zum Festlegen der Knochenplatte verwendet werden. Außerdem ist sichergestellt, daß jede Knochenschraube ihr eigenes Sicherungselement umfaßt. Insbesondere ist es günstig, daß das Sicherungselement an der Knochenschraube unverlierbar beweglich gelagert ist. Dadurch kann erreicht werden, daß insbesondere bei sehr kleinen Knochenschrauben, wie sie beispielsweise im cervicalen Bereich der Wirbelsäule eingesetzt werden, das Sicherungselement einfach und leicht von der Sicherungsstellung in die Entsicherungsstellung und umgekehrt überführbar ist. Außerdem kann es nicht in einem Operationsbereich verloren gehen. Damit in der Eingriffsstellung keine Spannungen an der Knochenschraube entstehen, die zu einer Beschädigung desselben unter Dauerbelastung führen können, ist es vorteilhaft, daß die Knochenschraube ohne Einwirkung äußerer Kräfte die Eingriffsstellung einnimmt. Dies ist wie beschrieben günstig, da beispielsweise bei Knochenschrauben, deren Kopf in der Eingriffsstellung aufgespreizt wird, der Kopf ständig unter Spannung steht und aufspreizbare Elemente unter Dauerbelastung abbrechen können. Um insbesondere auch eine unverlierbare Lagerung des Sicherungselements an der Knochenschraube zu ermöglichen, ist es vorteilhaft, daß der Schaft eine Sicherungselementaufnahme aufweist und daß das Sicherungselement in der Sicherungselementaufnahme gelagert ist. Damit das Sicherungselement unverlierbar an der Knochenschraube gehalten werden kann, ist es vorteilhaft, daß nach dem Einführen des Sicherungselements in die Sicherungselementaufnahme das Sicherungselement in axialer Richtung zwischen einem in distaler und einem in proximaler Richtung wirkenden Anschlag bewegbar ist. Das Sicherungselement kann also in axialer Richtung gegen den in proximaler Richtung wirkenden Anschlag bewegt werden, an welchem es beispielsweise in der Entsicherungsstellung anliegt. Des weiteren kann das Sicherungselement gegen den in distaler Richtung wirkenden Anschlag bewegt werden und an diesem anliegen, wenn es beispielsweise die Sicherungsstellung einnimmt. Gemäß der Erfindung ist vorgesehen, daß das Sicherungselement in einer Grundstellung der Knochenschraube, in welcher keine äußeren Kräfte auf die Knochenschraube wirken, in der Sicherungsstellung gehalten ist. Dies hat den Vorteil, daß ein Arzt nicht daran denken muß, die Knochenschraube zu verriegeln, da sie in einer Grundstellung die Sicherungsstellung einnimmt. Dadurch werden die Operationszeit verkürzt und Fehler vermieden. Ferner wird auch die Zahl der erforderlichen Instrumente verringert, da kein spezielles Instrument für das Sicherungselement benötigt wird. Um das Sicherungselement in der Sicherungsstellung zu halten, kann die Knochenschraube auch so ausgebildet sein, daß keine Kräfte auf das Sicherungselement in der Sicherungsstellung wirken, das Sicherungselement also kräftefrei gehalten ist. Vorteilhaft ist es, daß das Sicherungselement in der Sicherungsstellung unter Vorspannung gehalten ist. Zum Überführen des Sicherungselements von der Sicherungsstellung in die Entsicherungsstellung entgegen der Vorspannung ist daher eine bestimmte Lösekraft aufzubringen. Da das Sicherungselement unter Vorspannung gehalten ist, wird es automatisch wieder von der Entsicherungsstellung in die Sicherungsstellung zurücküberführt. Ein Arzt muß daher nicht jede Knochenschraube einzeln verriegeln, die Knochenschraube verriegelt durch die besondere Ausgestaltung sich selbst.

Ein besonders einfacher Aufbau der Knochenschraube ergibt sich, wenn sie rotationssymmetrisch oder im wesentlichen rotationssymmetrisch zur Längsachse ausgebildet ist.

Vorzugsweise weist der Kopf in der Eingriffsstellung eine maximale Außenabmessung quer zur Längsachse und in der Lösestellung eine gegenüber der maximalen Außenabmessung reduzierte Außenabmessung auf. Dies gestattet es, daß der Kopf mit einem Teil der Knochenplatte, beispielsweise der Knochenschraubenaufnahme, in der Eingriffsstellung in Eingriff und in der Lösestellung außer Eingriff bringbar. Der Kopf kann dabei eine beliebige Form aufweisen, beispielsweise mit einem eckigen oder runden Querschnitt quer zur Längsachse.

Insbesondere bei einem runden Querschnitt des Kopfes ist es vorteilhaft, wenn der Kopf in der Eingriffsstellung einen maximalen Außendurchmesser quer zur Längsachse und in der Lösestellung einen gegenüber dem maximalen Außendurchmesser reduzierten Außendurchmesser aufweist. Beispielsweise kann der Kopf ausgehend von der Eingriffsstellung zusammengedrückt werden zum Überführen der Knochenschraube in die Lösestellung oder aber auch von der Lösestellung aufgespreizt werden zum Überführen der Knochenschraube in die Eingriffsstellung. Daher wäre es denkbar, die Knochenschraube so auszubilden, daß sie ohne Einwirkung äußerer Kräfte entweder die Eingriffsstellung oder die Lösestellung einnimmt.

Eine Verbindung zwischen der Knochenschraube und der Knochenplatte kann auf besonders einfache Art hergestellt werden, wenn der Kopf ein erstes Rastelement trägt, welches in der Eingriffsstellung mit einem an der Knochenplatte angeordneten zweiten Rastelement verrastbar ist. Die beiden Rastelemente können also in der Eingriffsstellung in Eingriff stehen, insbesondere formschlüssig ineinandergreifen.

Die Konstruktion der Knochenschraube läßt sich besonders einfach gestalten, wenn das erste Rastelement eine sich in Umfangsrichtung erstreckende, radial geöffnete Ringnut oder ein sich in Umfangsrichtung erstreckender, radial nach außen abstehender Ringvorsprung ist. Die Ringnut kann beispielsweise einen korrespondierenden Vorsprung oder korrespondierende Vorsprünge an der Knochenplatte aufnehmen. Der abstehende Ringvorsprung kann mit einer korrespondierenden Nut an der Knochenplatte in Eingriff gebracht werden zum Herstellen einer Verbindung zwischen der Knochenschraube und der Knochenplatte.

Vorteilhaft ist es, wenn zum Halten des Sicherungselements in der Sicherungsstellung mindestens ein Halteelement vorgesehen ist, wenn es sich einerseits am Schaft und andererseits am Sicherungselement abstützt. Insbesondere kann das Sicherungselement in einem Hohlraum oder einer Ausnehmung des Schafts angeordnet sein, in welchem es zudem geschützt ist.

Definierte Haltekräfte in unterschiedlichen Stellungen des Sicherungselements lassen sich erzeugen, wenn das Halteelement ein elastisches Element ist.

Besonders einfach wird der Aufbau der Knochenschraube, wenn das Halteelement eine Schraubenfeder oder eine Tellerfeder oder Paket von Tellerfedern ist. Eine gewünschte Haltekraft, mit welcher das Sicherungselement in der Sicherungsstellung gehalten oder auch in dieser unter Vorspannung gehalten ist, kann auf einfache Weise eingestellt werden.

Gemäß einer bevorzugten Ausführungsform der Erfindung kann ferner vorgesehen sein, daß das Halteelement derart ausgebildet ist, daß eine äußere Abmessung und/oder ein Volumen des Halteelements durch Änderung mindestens einer Zustandsgröße in einer Umgebung der Knochenschraube änderbar ist. Beispielsweise kann in der Umgebung der Knochenschraube ein Druck erhöht werden, was zur Folge hat, daß das Volumen des Halteelements vergrößert oder verkleinert wird. Durch die Änderung der äußeren Abmessung und/oder des Volumens des Halteelements kann das Sicherungselement von der Entsicherungsstellung in die Sicherungsstellung überführt werden und/oder umgekehrt.

Günstig ist es, wenn die mindestens eine Zustandgröße eine Umgebungstemperatur, ein Umgebungsdruck, ein osmotischer Druck oder eine Feuchtigkeit der Umgebung ist. Dies gestattet es durch Änderung der genannten oder auch weiterer physikalischer Größen in der Umgebung der Knochenschraube, den Zustand des Halteelements zu ändern, insbesondere eine äußere Abmessung und/oder ein Volumen desselben.

Günstigerweise ist das Halteelement aus einem Memorymetall hergestellt. Insbesondere ist es so beispielsweise möglich, das Sicherungselement von der Sicherungsstellung in die Entsicherungsstellung durch Abkühlen oder Erwärmen zu überführen und umgekehrt wiederum durch Erwärmen oder Abkühlen.

Ferner kann das Sicherungselement auf einfache Weise bewegt werden, wenn das Halteelement durch ein Fluid inflatierbar ist. Beispielsweise kann das Fluid Luft, Druckluft oder eine körperverträgliche Flüssigkeit sein.

Um insbesondere Änderungen eines osmotischen Drucks ausnutzen zu können, um das Halteelement in seiner Größe zu ändern, ist es günstig, wenn das Halteelement einen Innenraum aufweist, welcher über eine semipermeable Membran mit einer Umgebung der Knochenschraube in Fluidverbindung steht. Dies gestattet es beispielsweise, Wasser in den Innenraum des Halteelement eindifundieren zu lassen in Abhängigkeit einer Lösungsmittelkonzentration in der Umgebung der Knochenschraube.

Eine Sicherung der Knochenschraube in der Eingriffsstellung läßt sich besonders leicht erreichen, wenn das Sicherungselement parallel zur Längsachse verschiebbar am Schaft gelagert ist.

Die Knochenschraube ist besonders einfach herzustellen, wenn die Sicherungselementaufnahme ein Sackloch ist. Das Sicherungselement kann dann zunächst in die Sicherungselementaufnahme durch eine Öffnung des Sacklochs eingeführt werden, jedoch nicht auf der anderen Seite der Ausnehmung wieder aus der Knochenschraube austreten. Vorzugsweise ist das Sackloch so angeordnet, daß eine Öffnung desselben im Bereich des Kopfs der Knochenschraube angeordnet ist.

Vorteilhafterweise ist das Sicherungselement drehformschlüssig in der Sicherungselementaufnahme gehalten. Dies gestattet es, daß eine Kraft auf die Knochenschraube zum Eindrehen derselben in ein Knochenteil über das Sicherungselement eingeleitet und durch die drehformschlüssige Ausbildung der Sicherungselementaufnahme auf den Schaft der Knochenschraube übertragen werden kann. So wird es insbesondere möglich, einen Werkzeugadapter für ein Einschraubwerkzeug der Knochenschraube am Sicherungselement selbst vorzusehen.

Die Stabilität der Knochenschraube wird insgesamt erhöht, wenn das Sicherungselement einen Sicherungselementabschnitt aufweist, welcher in Form eines Außenvielkant ausgebildet ist, und wenn die Sicherungselementaufnahme einen Sicherungselementaufnahmeabschnitt aufweist, welcher in Form eines zum Außenvielkant korrespondierenden Innenvielkants ausgebildet ist. Beispielsweise kann der Außenvielkant als Vierkant, Sechskant oder Achtkant ausgebildet werden. Eine Kraftübertragung in Folge einer Rotation des Sicherungselements auf den Schaft der Knochenschraube kann so optimiert werden.

Um auf einfache Weise einen Übergang der Knochenschraube von der Eingriffsstellung in die Lösestellung und umgekehrt zu realisieren, ist es vorteilhaft, wenn das erste Rastelement an der Knochenschraube in radialer Richtung bewegbar gelagert ist. Es kann also von der Längsachse in radialer Richtung weg oder auf diese zu bewegt werden. Die Rastelemente können insbesondere verschiebbar oder verschwenkbar an der Knochenschraube gelagert sein.

Eine Verbindung zwischen der Knochenschraube und der Knochenplatte läßt sich auf einfache Weise herstellen, wenn der Kopf eine Mehrzahl quer zur Längsachse bewegbarer Verriegelungselemente umfaßt, welche in der Eingriffsstellung mit der Knochenplatte in Eingriff stehen.

Grundsätzlich wäre es denkbar, anstelle einer Rastverbindung zwischen der Knochenschraube und der Knochenplatte auch eine andere Verbindung vorzusehen, beispielsweise eine Bajonettverbindung. Wird jedoch eine Rastverbindung gewünscht, dann ist es günstig, wenn die Verriegelungselemente das erste Rastelement tragen. Das erste Rastelement kann dann infolge einer Bewegung des Verriegelungselements beziehungsweise der Verriegelungselemente mit dem zweiten Rastelement an der Knochenplatte in Eingriff gebracht werden.

Weist die Knochenschraube eine Sicherungselementaufnahme auf, dann ist zumindest ein Teil der Knochenschraube hülsenartig ausgebildet. Vorteilhaft ist es dann, wenn der Kopf lappenartige, durch sich parallel zur Längsachse erstreckende Schlitze getrennte Wandabschnitte aufweist und wenn die Wandabschnitte die Verriegelungselemente bilden. Zur Herstellung der Verriegelungselemente muß also beispielsweise nur der Kopf der Knochenschraube geschlitzt werden. Vorteilhafterweise werden vier, fünf oder sechs Verriegelungselemente vorgesehen, die durch vier, fünf oder sechs symmetrisch über einen Umfang der Knochenschraube verteilte Schlitze ausgebildet werden können.

Günstig ist es, wenn die Verriegelungselemente in einer Grundstellung, in welcher keine äußeren Kräfte auf die Knochenschraube wirken, die Eingriffsstellung einnehmen. Die Verriegelungselemente müssen also nur zum Überführen der Knochenschraube von der Eingriffsstellung in die Lösestellung bewegt werden und stehen nur für diese kurze Zeit unter Spannung. In der Eingriffsstellung sind sie jedoch im wesentlichen spannungsfrei, so daß keine Gefahr besteht, daß die Verriegelungselemente unter Dauerbelastung abbrechen können.

Zum Überführen der Knochenschraube von der Eingriffsstellung in die Lösestellung kann vorgesehen sein, daß die Verriegelungselemente radial in Richtung auf die Längsachse hin bewegt werden. Daher sind günstigerweise zum Einführen des Sicherungselements in die Sicherungselementaufnahme die Verriegelungselemente von der Eingriffsstellung radial nach außen bewegbar. Dies bedeutet mit anderen Worten, daß das Sicherungselement beim Einführen in die Sicherungselementaufnahme die Verriegelungselemente und damit beispielsweise einen Kopf der Knochenschraube aufspreizt. Nach Einführen des Sicherungselements in die Sicherungselementaufnahme können die Verriegelungselemente dann wieder in ihre ursprüngliche Form zurückgehen, in welche sie die Eingriffsstellung einnehmen. Das Sicherungselement ist bei dieser Ausführungsform dann unverlierbar in der Sicherungselementaufnahme gehalten. Zum Entfernen des Sicherungselements aus der Sicherungselementaufnahme müssen dann die Verriegelungselemente wieder radial nach außen verschwenkt werden, um die Öffnung der Sicherungselementaufnahme so weit freizugeben, daß das Sicherungselement aus dieser entnommen werden kann.

Um das Sicherungselement unverlierbar an der Knochenschraube zu halten, ist es günstig, wenn eine zweite Rastvorrichtung umfassend ein drittes und ein viertes, mit dem dritten Rastelement zusammenwirkendes Rastelement vorgesehen ist, wenn das Sicherungselement und der Kopf jeweils ein Rastelement tragen und wenn die zweite Rastvorrichtung eine Raststellung nach dem Einführen des Sicherungselements in die Sicherungselementaufnahme einnimmt. Eine Verrastung ergibt sich also vorzugsweise dann, wenn das Sicherungselement in die Sicherungselementaufnahme eingeführt ist. Zum Lösen der Rastverbindung muß mindestens eines der beiden Rastelemente relativ zum anderen bewegt werden. Geschieht dies nicht, dann ist das Sicherungselement unverlierbar in der Sicherungselementaufnahme gehalten.

Ein besonders einfacher Aufbau der Knochenschraube ergibt sich, wenn das dritte Rastelement eine in proximaler Richtung wirkende, radial nach außen abstehende Rastnase ist und wenn das vierte Rastelement eine radial nach innen vorstehende, in distaler Richtung wirkende Rastkante ist. Die Rastnase und die Rastkante können sich in der Raststellung zum Ausbilden einer Rastverbindung hintergreifen und so das Sicherungselement in der Sicherungselementaufnahme sichern.

Eine Relativbewegung des dritten Rastelements und des vierten Rastelements läßt sich auf einfache Weise dadurch realisieren, daß die Verriegelungselemente das vierte Rastelement tragen. Dadurch kann bei einer Bewegung der Verriegelungselemente das vierte Rastelement relativ zum dritten bewegt und zu diesem in Eingriff oder außer Eingriff gebracht werden.

Vorteilhafterweise bilden das dritte oder das vierte Rastelement den proximalen Anschlag und liegen in der Sicherungsstellung aneinander an. Insbesondere ist es denkbar, daß das Halteelement das Sicherungselement in proximaler Richtung drückt, so daß das dritte oder vierte Rastelement am proximalen Anschlag, welcher in distaler Richtung wirkt, anliegen.

Um das Zusammensetzen der Knochenschraube zu erleichtern und insbesondere Instrumente zum Zusammenbauen der Knochenschraube überflüssig zu machen, ist es vorteilhaft, wenn sich an das dritte oder das vierte Rastelement eine Aufgleitfläche anschließt, an welcher das jeweils andere Rastelement beim Einführen des Sicherungselements in die Sicherungselementaufnahme aufgleiten kann. Beispielsweise kann sich die Aufgleitfläche ausgehend vom dritten, am Sicherungselement angeordneten Rastelement in distaler Richtung erstrecken, so daß das vierte, am Kopf der Knochenschraube angeordnete Rastelement beim Einführen des Sicherungselements in die Sicherungselementaufnahme aufgleiten kann und dadurch in radialer Richtung nach außen bewegt wird, bis es das dritte Rastelement hintergreift, wodurch es wieder in radialer Richtung auf die Längsachse hin bewegbar ist. Es läßt sich so das Sicherungselement in der Sicherungselementaufnahme sichern.

Die Stabilität der Knochenschraube läßt sich erhöhen, wenn das Sicherungselement in Form eines Kopfbolzens ausgebildet ist mit einem Bolzenschaft und einem Bolzenkopf.

Der Aufbau des Sicherungselements wird besonders einfach, wenn der Bolzenschaft den Sicherungselementabschnitt bildet, welcher in Form eines Außenvielkant ausgebildet ist.

Um das Sicherungselement auf einfache Weise unverlierbar in der Sicherungselementaufnahme beweglich zu halten, ist es vorteilhaft, wenn der Bolzenkopf das dritte Rastelement trägt. Beispielsweise kann das dritte Rastelement in Form eines radial nach außen abstehenden ringförmigen Vorsprungs ausgebildet sein, welcher eine sich quer zur Längsachse in proximaler Richtung erstreckende Anschlagsfläche bildet, an welche sich eine sich in distaler Richtung konisch verjüngende Aufgleitfläche anschließt.

Um die Knochenschraube auf einfache Weise in ein Knochenteil einschrauben zu können, ist es günstig, wenn die Knochenschraube einen Werkzeugelementadapter aufweist, welcher mit einem Einschraubwerkzeug zum Einschrauben der Knochenschraube in Eingriff bringbar ist.

Vorzugsweise trägt das Sicherungselement den Werkzeugadapter. Dies hat den Vorteil, daß mit dem Einschraubwerkzeug gleichzeitig zwei Funktionen ausübbar sind. Zum einen kann das Einschraubwerkzeug bei bestimmten Ausführungsformen das Sicherungselement von der Sicherungsstellung in die Entsicherungsstellung überführen, beispielsweise auch entgegen der von einem Halteelement erzeugten Vorspannung. Zum anderen kann mit dem Einschraubwerkzeug die gesamte Knochenschraube eingedreht werden, beispielsweise dann, wenn das Sicherungselement drehformschlüssig in der Sicherungselementaufnahme gehalten ist.

Grundsätzlich wäre es denkbar, daß der Werkzeugadapter in Form eines Vorsprungs ausgebildet ist. Günstig ist es jedoch, wenn der Werkzeugadapter eine Ausnehmung ist. Dabei kann es vorteilhaft sein, wenn die Ausnehmung in Form eines Schlitzes, eines Vielkants oder einer sternförmigen Vertiefung ausgebildet ist.

Zur Erhöhung der Stabilität der Knochenschraube ist es vorteilhaft, wenn der Kopf und der Schaft einstückig ausgebildet sind.

Gemäß einer bevorzugten Ausführungsform der Erfindung kann vorgesehen sein, daß der Schaft mit einem Außengewinde versehen ist. Insbesondere kann das Außengewinde ein selbstschneidendes Knochengewinde sein. Durch das Außengewinde wird ein sicherer Halt der Knochenschraube im Knochenteil gewährleistet.

Die eingangs gestellte Aufgabe wird erfindungsgemäß auch bei einem Implantatsystem umfassend mindestens eine Knochenplatte mit mindestens einer Knochenschraubenaufnahme und mindestens eine Knochenschraube, welche mit der Knochenschraubenaufnahme in einer Eingriffsstelllung in Eingriff bringbar ist zum lösbaren Verbinden der Knochenschraube mit der Knochenplatte, dadurch gelöst, daß die Knochenschraube eine der oben beschriebenen Knochenschrauben ist. Es ergeben sich dadurch die bereits dargelegten Vorteile bei der Verbindung von Knochenteilen mit der Knochenplatte des Implantatsystems, wobei die Kochenplatte mit der mindestens einen Knochenschraube an dem Knochenteil festgelegt werden kann.

Die nachfolgende Beschreibung bevorzugter Ausführungsformen der vorliegenden Erfindung dienen im Zusammenhang mit der Zeichnung der näheren Erläuterung. Es zeigen:
- Figur 1:: eine perspektivische Ansicht eines Implantatsystems umfassend eine Knochenplatte und zwei Knochenschrauben;
- Figur 2:: eine perspektivische Ansicht eines proximalen Endes einer Knochenschraube aus Figur 1 vor dem Einsetzen eines Sicherungselements;
- Figur 3:: eine teilweise geschnittene Seitenansicht eines proximalen Endes einer Knochenschraube aus Figur 1 in der Eingriffsstellung;
- Figur 4:: eine teilweise geschnittene Seitenansicht der Knochenschraube mit dem Sicherungselement in der Eingriffsstellung;
- Figur 5:: eine Ansicht ähnlich Figur 4, wobei die Knochenschraube die Lösestellung einnimmt;
- Figur 6:: eine teilweise geschnittene Seitenansicht durch ein zweites Ausführungsbeispiel einer Knochenschraube in der Lösestellung; und
- Figur 7:: eine Ansicht ähnlich Figur 6 eines dritten Ausführungsbeispiels einer Knochenschraube.

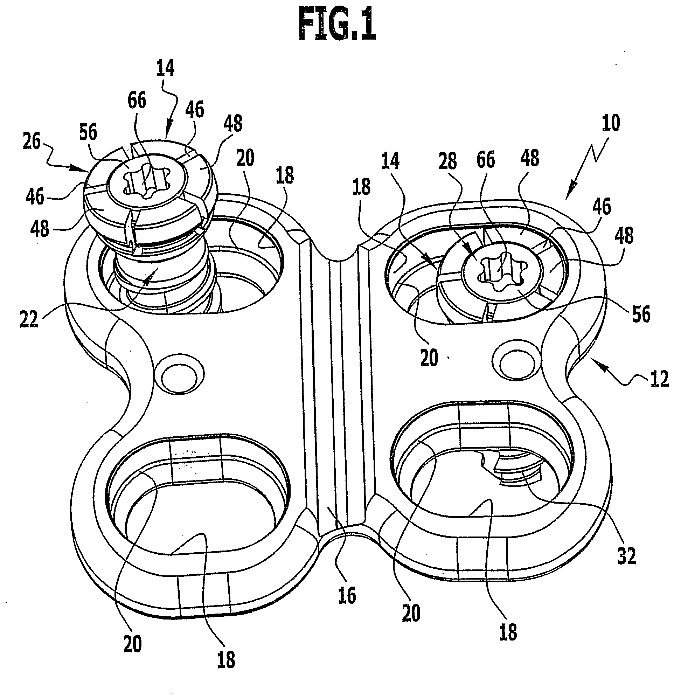

In Figur 1 ist ein insgesamt mit dem Bezugszeichen 10 versehenes Implantatsystem umfassend eine Knochenplatte 12 und zwei selbstverriegelnde Knochenschrauben 14 dargestellt.

Die in Figur 1 dargestellte Knochenplatte 12 dient in erster Linie der Illustration, sie stellt keine Beschränkung der vorliegenden Erfindung dar. Grundsätzlich wären alle Arten von Knochenplatten im Zusammenhang mit der vorliegenden Erfindung denkbar, die zumindest eine Durchbrechung für eine Knochenschraube aufweisen.

Die Knochenplatte 12 weist eine flache, kleeblattartige Form auf mit allseits abgerundeten Außenkanten. Etwa mittig ist eine nutartige Vertiefung 16 vorgesehen, die quasi jeweils zwei Blattpaare der Knochenplatte 12 voneinander trennt. Insgesamt ist die Knochenplatte 12 einstückig ausgebildet. Im Bereich jeder Ecke der Knochenplatte 12 ist eine langlochartige Durchbrechung 18 vorgesehen, wobei Längsrichtungen der Durchbrechungen 18 im wesentlichen parallel zueinander ausgerichtet sind. Eine maximale Erstreckung der Durchbrechungen 18 ist quer zur Längsrichtung der Vertiefung 16 orientiert, so daß eine minimale innere Abmessung der Durchbrechungen 18 im wesentlichen parallel zur Längsrichtung der Vertiefung 16 vorgegeben ist. Innenränder 20 der Durchbrechungen 18 sind in Richtung auf ein Zentrum der jeweiligen Durchbrechung 18 hin konkav gekrümmt, so daß sowohl an der Ober- als auch an der Unterseite der Knochenplatte 12 die Durchbrechung 18 begrenzende Ränder radial nach innen vorstehen. Die Durchbrechungen 18 bilden so mit ihren konkaven Rändern 20 im wesentlichen hohlkalottenförmige Hinterschneidungen an der Knochenplatte 20.

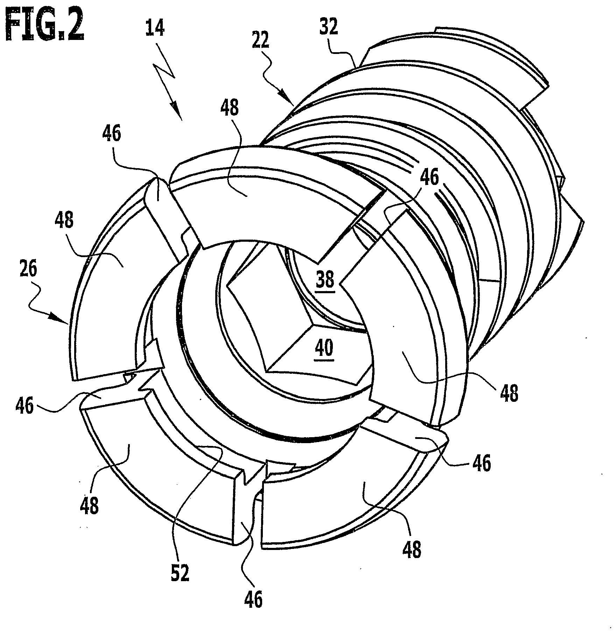

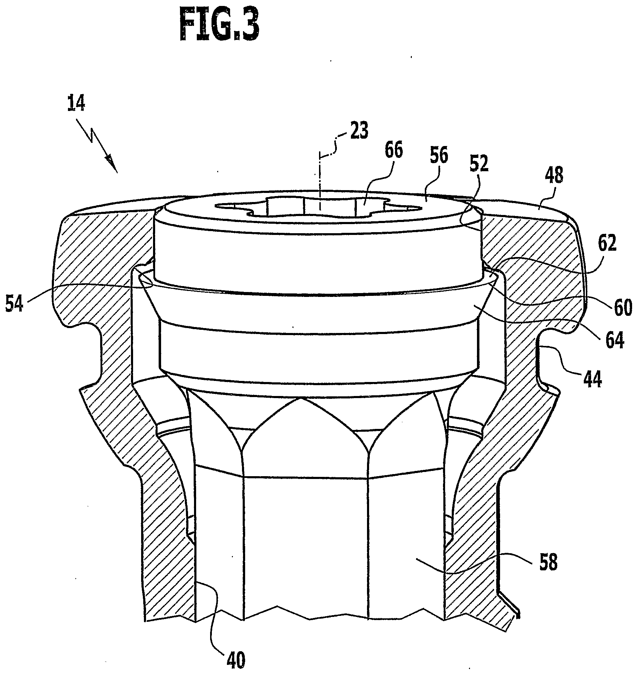

Zum Festlegen der Knochenplatte 12 an nicht dargestellten Knochenteilen dienen die Knochenschrauben 14, die in Form eines ersten Ausführungsbeispiels im Zusammenhang mit den Figuren 1 bis 5 nachfolgend näher erläutert werden.

Die Knochenschraube 14 ist im wesentlichen dreiteilig ausgebildet. Sie umfaßt einen Schaft 22, welcher eine Längsachse 23, distalseitig eine Schraubenspitze 24 und proximalseitig einen Schraubenkopf 26 definiert, sowie ein Sicherungselement in Form eines Verriegelungspins 28 und einer als Halteelement dienenden Schraubenfeder 30. Der Schaft 22 ist ausgehend von seiner Schraubenspitze 24 über etwa dreiviertel seiner Länge mit einem Außengewinde 32 versehen, welches vorzugsweise selbstschneidend ausgebildet ist. Eine Außenkontur oder Einhüllende des Schraubenkopfes 26 ist im wesentlichen kalottenförmig ausgebildet und an die durch den Innenrand 20 gebildete Hinterschneidung der Durchbrechung 18 angepaßt, wodurch eine polyaxiale Anpassung des Schraubenkopfes 26 in der hinterschnittenen Durchbrechung 18 ermöglicht wird.

Ausgehend von seinem proximalen Ende ist der Schaft 22 mit einem eine Sicherungselementaufnahme bildenden Sackloch 34 versehen, dessen distalseitiger Boden 36 in Richtung auf das proximale Ende der Knochenschraube 14 hin weist. Ausgehend vom Boden 36 erstreckt sich auf etwa einem Drittel der Gesamtlänge des Sacklochs 34 ein Hohlzylinderabschnitt 38, in welchen die Schraubenfeder 30 eingesetzt ist, die sich mit ihrem distalseitgen Ende am Boden 36 abstützt. An den Hohlzylinderabschnitt 38 schließt sich ein Innensechskantabschnitt 40 an, welcher in etwa ein mittleres Drittel des Sacklochs 34 bildet. Der Innendurchmesser des Hohlzylinderabschnitts 38 ist etwas kleiner als eine Innenabmessung des Innensechskantabschnitts 40, so daß im Übergangsbereich zwischen dem Hohlzylinderabschnitt 38 und dem Innensechskantabschnitt 40 ein distaler, durch eine in proximaler Richtung weisende Ringfläche gebildeter Anschlag 42 gebildet wird.

In etwa das proximale Drittel des Schafts 22 bildet den Schraubenkopf 26, welcher einen gegenüber den mit dem Außengewinde 32 versehenen Schaftabschnitt vergrößerten Außendurchmesser aufweist. Auf einer Außenseite ist der Schraubenkopf 26 mit einer umlaufenden Ringnut 44 versehen. Um den Schraubenkopf 26 in seinem Außendurchmesser variieren zu können, ist er symmetrisch über seinen Umfang mit fünf sich parallel zur Längsachse 23 erstreckenden Schlitzen 46 versehen, so daß insgesamt fünf, Verriegelungselemente bildende Segmente 48 des Schraubenskopfs 26 ausgebildet werden. Die Segmente 48 bilden lappenartige Hülsenabschnitte, welche in radialer Richtung, also in Richtung auf die Längsachse 23 hin oder von dieser weg, verschwenkbar sind. Eine gewünschte Verschwenkbarkeit kann über die Tiefe der Ringnut 44 eingestellt werden, die eine Schwächung bildet und eine Beweglichkeit der Segmente 48 erleichtert. Eine Öffnung 50 des Sacklochs 34 ist mit einem radial nach innen auf die Längsachse 23 hin vorstehenden Ringflansch 52 versehen, welcher ebenso wie die Ringnut 44 durch die Schlitze 46 unterbrochen ist. Der Ringflansch 52 bildet eine Innendurchmesserverengung des Sacklochs 34. Ferner weist er eine in distaler Richtung weisende ringförmige Anschlagfläche 54 auf, welche einen proximalseitigen, in distaler Richtung wirkenden Anschlag bildet.

Ausgehend vom Innensechskantabschnitt 40 erweitert sich ein Innendurchmesser des Sacklochs 34 bis auf einen maximalen Innendurchmesser hin, welcher sich innen im Bereich der Ringnut 44 bis an die Anschlagfläche 54 hin erstreckt.

Der Verriegelungspin 28 ist insgesamt in Form eines Kopfbolzens mit einem Kopf 56 und einem in Form eines zum Innensechskantabschnitt 40 korrespondierenden, in Form eines Außensechskants ausgebildeten Bolzenschaft 58 geformt. Der im Außendurchmesser gegenüber dem Bolzenschaft 58 größere Kopf 56 weist eine sich in Umfangsrichtung erstreckende, radial nach außen abstehende Schnapplippe 60 auf, welche eine ringförmige Anschlagfläche 62 umfaßt, die in proximaler Richtung weist und eine sich vom äußeren Rand der Anschlagfläche 62 in distaler Richtung erstreckende, sich konisch verjüngende Aufgleitfläche 64.

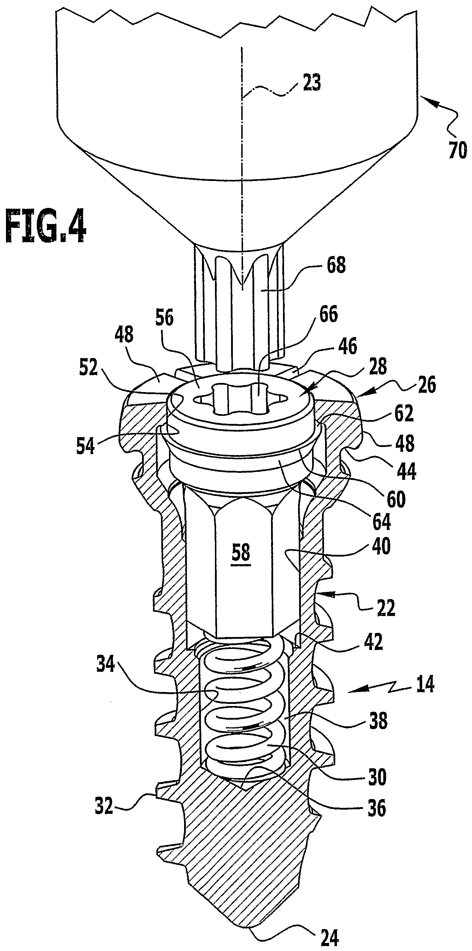

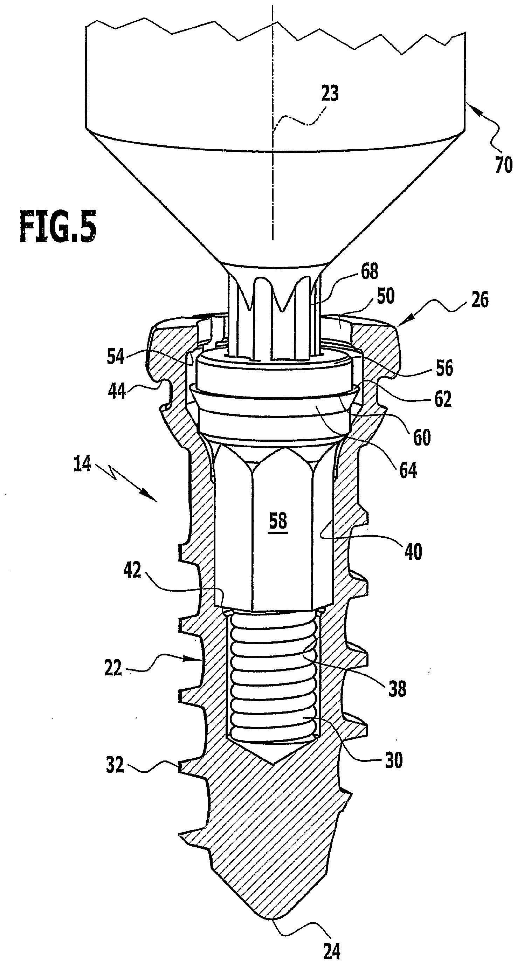

Am Kopf ist eine in proximaler Richtung weisende, sacklochartige, sternförmige Vertiefung vorgesehen, welche als Werkzeugaufnahme 66 dient zur Aufnahme einer korrespondierenden Werkzeugspitze eines Einschraubwerkzeugs 70, dessen distales Ende in den Figuren 4 und 5 dargestellt ist.

Zum Zusammensetzen der dreiteiligen Knochenschraube 14 wird wie folgt vorgegangen. Zuerst wird die Schraubenfeder 30 durch die Öffnung 50 hindurch in das Sackloch 34 eingeführt, bis ein distales Ende der Schraubenfeder 30 am Boden 36 anliegt. Als nächstes wird der Verriegelungspin 28 mit seinem Bolzenschaft durch die Öffnung 50 hindurchgesteckt, bis der Ringflansch 52 an der Aufgleitfläche 64 anschlägt. Zum vollständigen Einführen des Verriegelungspins 28 in das Sackloch 34 müssen nun die Segmente 48 radial nach außen aufgespreizt werden. Dies geschieht durch Kraftausübung auf den Verriegelungspin 28 in distaler Richtung. Der Ringflansch 52 an den Segmenten 48 gleitet dann an der Aufgleitfläche 64 auf, so daß die Segmente 48 radial nach außen verschwenkt werden und hinter die Schnapplippe 60 radial nach innen zurückfedern, sobald die Anschlagfläche 62 die Anschlagfläche 54 am Ringflansch 52 hintergreift. Ein proximales Ende der Schraubenfeder 30 liegt am distalen Ende des Bolzenschafts 58 an und drückt den Verriegelungspin 48 in proximaler Richtung und hält so die Anschlagflächen 54 und 62 gegeneinander in Anlage. Die Knochenschraube 14 nimmt dann, wie in den Figuren 1, 3 und 4 dargestellt, ihre Eingriffsstellung ein.

Zum Einschrauben der Knochenschraube 14 in ein nicht dargestelltes Knochenteil und zum Sichern der Knochenplatte 12 an derselben wird die Knochenschraube 14 mit Ihrem mit dem Außengewinde 32 versehenen Schaft 22 zunächst durch die Durchbrechung 18 hindurchgesteckt. Greift die Werkzeugspitze 68 in die Werkzeugaufnahme 66 ein, so wird der Verriegelungspin 28 entgegen der Federkraft der Schraubenfeder 30 in distaler Richtung bewegt, bis das distale Ende des Bolzenschafts 58 am Anschlag 42 anschlägt. Der Verriegelungspin 28 nimmt dann die in Figur 5 dargestellte Entsicherungsstellung ein.

Durch die drehformschlüssige Ausbildung des Innensechskantabschnitts 40 und des Bolzenschafts 58 kann ein von der Werkzeugspitze 68 in den Kopf 56 eingeleitetes Drehmoment auf den Schaft 22 der Knochenschraube 14 übertragen werden. Die Knochenschraube 14 kann immer tiefer in das Knochenteil eingeschraubt werden, und zwar soweit, bis der Schraubenkopf 26 mit den verschwenkbaren Segmenten 48 am oberen ringförmigen Rand der Durchbrechung 18 anschlägt. Die Segmente 48 gleiten beim weiteren Einschrauben der Knochenschraube 14 am oberen Rand auf und werden radial nach innen verschwenkt, bis ein proximales Ende der Knochenschraube 14, also der Schraubkopf 26, ganz in die hinterschnittene Durchbrechung 18 eintauchen kann. Dann federn die Segmente 48 radial nach außen zurück und nehmen wieder ihre Eingriffsstellung ein. Der Schraubenkopf 26 ist so in der hinterschnittenen Durchbrechung 18 gehalten, aber noch nicht gesichert.

Das Eintauchen des Schraubenkopfes 26 in die hinterschnittene Durchbrechung 18 ist nur möglich, wenn der Verriegelungspin 28 die in Figur 5 dargestellte Entsicherungsstellung einnimmt. Sitzt die Knochenschraube 14 in gewünschter Weise im Knochen, entfernt ein Operateur das Einschraubwerkzeug 70. Durch die von der Schraubenfeder 30 ausgeübte Rückstellkraft auf den Verriegelungspin 28 wird dieser in proximaler Richtung bewegt, bis die Anschlagfläche 62 der Schnapplippe 60 wieder an der Anschlagfläche 54 anschlägt. Der Kopf 56 des Verriegelungspins 28 sitzt dann bündig zwischen den Segmenten 48 und bildet einen flachen, proximalseitigen Abschluß der Knochenschraube 14.

Ohne Einwirkung äußerer Kräfte nimmt die Knochenschraube 14 die Eingriffsstellung ein und wird durch den mittels der Schraubenfeder 30 in der Sicherungsstellung gehaltenen Verriegelungspin 28 in der Eingriffsstellung gesichert.

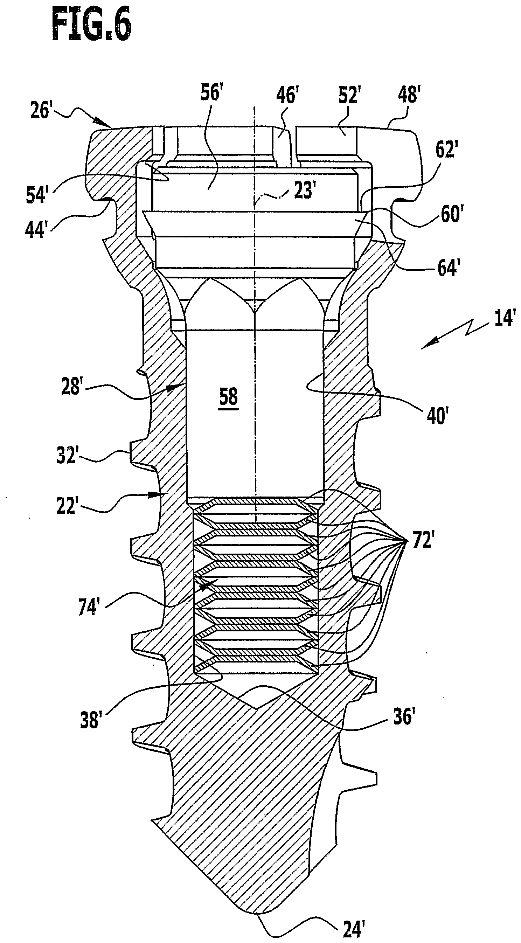

In Figur 6 ist eine insgesamt mit dem Bezugszeichen 14' versehene zweite Ausführungsform einer Knochenschraube dargestellt. Die Knochenschraube 14' unterscheidet sich von der in den Figuren 1 bis 5 dargestellten Knochenschraube 14 dadurch, daß anstelle der Schraubenfeder 30 ein insgesamt elf Tellerfedern 72' umfassendes Tellerfedernpaket 74' vorgesehen ist, welches sich distalseitig am Boden 36' und proximalseitig am distalen Ende des Bolzenschafts 58' abstützt. Alle übrigen Teile und Elemente der Knochenschraube 14' stimmen mit denen der Knochenschraube 14 überein, so daß dieselben Teile auch mit denselben Bezugsziffern versehen sind, die Figur 6 jedoch zusätzlich einen hochgestellten Strich (') tragen. Die Funktionsweise der Knochenschraube 14' entspricht in vollem Umfang der im Zusammenhang mit den Figuren 1 bis 5 beschriebenen Knochenschraube 14.

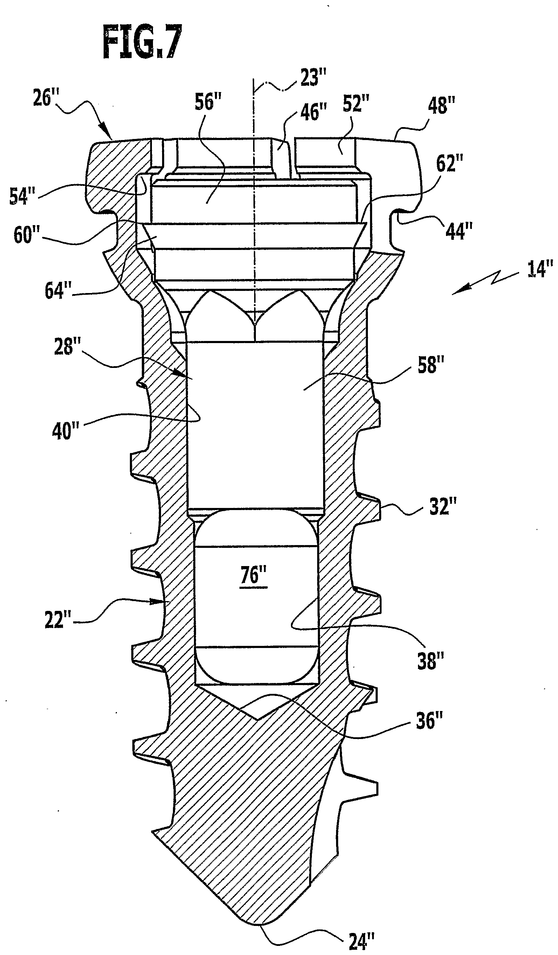

Ein drittes Ausführungsbeispiel einer insgesamt mit dem Bezugszeichen 14" versehenen Knochenschraube ist in Figur 7 dargestellt. Anstelle der Schraubenfeder 30 der Knochenschraube 14 ist bei der Knochenschraube 14" ein Haltekörper 76" vorgesehen. Alle anderen Teile und Elemente der Knochenschraube 14" stimmen mit denen der Knochenschraube 14 überein, so daß identische Bezugszeichen verwendet, jedoch mit zwei zusätzlichen Strichen (") versehen sind.

Der Haltekörper 76" ist im dargestellten Ausführungsbeispiel in seinem Volumen veränderbar. Hierzu weist er eine Hülle auf, welche ein dehn- oder formänderndes Element umgibt, welches aus einem Memorymetall hergestellt ist. Durch Temperaturänderungen an der Knochenschraube dehnt sich das Element aus oder zieht sich zusammen, so daß der Verriegelungspin 28" von der in Figur 7 dargestellten Entsicherungsstellung in die Sicherungsstellung überführt werden kann, in welcher die Anschlagflächen 54" und 62" aneinander anliegen.

## Patentansprüche

1. Knochenschraube (14) mit einem eine Längsachse (23) definierenden Schaft (22) und mit einem Kopf (26), welcher mit einer Knochenschraubenaufnahme (18) einer Knochenplatte (12) in Eingriff bringbar ist zum lösbaren Verbinden der Knochenschraube (14) mit der Knochenplatte (12), wobei ein Sicherungselement (28) zum Sichern einer Verbindung der Knochenschraube (14) und der Knochenplatte (12) vorgesehen ist, wobei die Knochenschraube (14) von einer Eingriffsstellung, in welcher die Knochenschraube (14) an der Knochenplatte (12) gehalten ist, in eine Lösestellung bringbar ist, in welcher die Knochenschraube (14) von der Knochenplatte (12) lösbar ist, wobei das Sicherungselement (28) von einer Entsicherungsstellung, in welcher die Knochenschraube (14) in die Lösestellung bringbar ist, in eine Sicherungsstellung zum Sichern der Verbindung zwischen der Knochenschraube (14) und der Knochenplatte (12) bringbar ist, in welcher die Knochenschraube (14) die Eingriffsstellung einnimmt, wobei das Sicherungselement (28) an der Knochenschraube (14) beweglich gelagert ist und die Knochenschraube (14) in einer Grundstellung, in welcher keine äußeren Kräfte auf sie wirken, die Eingriffsstellung einnimmt, wobei der Schaft (22) eine Sicherungselementaufnahme (34) aufweist und das Sicherungselement (28) in der Sicherungselementaufnahme (34) gelagert ist, wobei nach dem Einführen des Sicherungselements (28) in die Sicherungselementaufnahme (34) das Sicherungselement (28) in axialer Richtung zwischen einem in distaler und einem in proximaler Richtung wirkenden Anschlag (42, 54) bewegbar ist und wobei das Sicherungselement (28) in der Grundstellung der Knochenschraube (14) in der Sicherungsstellung gehalten ist, **dadurch gekennzeichnet, daß** das Sicherungselement (28) in der Sicherungsstellung unter Vorspannung gehalten ist.

2. Knochenschraube nach Anspruch 1, **dadurch gekennzeichnet, daß** die Knochenschraube (14) rotationssymmetrisch oder im wesentlichen rotationssymmetrisch zur Längsachse (23) ausgebildet ist.

3. Knochenschraube nach einem der voranstehenden Ansprüche, **dadurch gekennzeichnet, daß** der Kopf (26) in der Eingriffsstellung eine maximale Außenabmessung quer zur Längsachse (23) und in der Lösestellung eine gegenüber der maximalen Außenabmessung reduzierte Außenabmessung aufweist.

4. Knochenschraube nach Anspruch 3, **dadurch gekennzeichnet, daß** der Kopf (26) in der Eingriffsstellung einen maximalen Außendurchmesser quer zur Längsachse (23) und in der Lösestellung eine gegenüber dem maximalen Außendurchmesser reduzierten Außendurchmesser aufweist.

5. Knochenschraube nach einem der voranstehenden Ansprüche, **dadurch gekennzeichnet, daß** der Kopf (26) ein erstes Rastelement (48) trägt, welches in der Eingriffsstellung mit einem an der Knochenplatte (12) angeordneten zweiten Rastelement (20) verrastbar ist.

6. Knochenschraube nach Anspruch 5, **dadurch gekennzeichnet, daß** das erste Rastelement (48) eine sich in Umfangsrichtung erstreckende, radial geöffnete Ringnut oder ein sich in Umfangsrichtung erstreckender, radial nach außen abstehender Ringvorsprung ist.

7. Knochenschraube nach einem der voranstehenden Ansprüche, **dadurch gekennzeichnet, daß** zum Halten des Sicherungselements (28) in der Sicherungsstellung mindestens ein Halteelement (30; 74'; 76") vorgesehen ist, welches sich einerseits am Schaft (22) und andererseits am Sicherungselement (28) abstützt.

8. Knochenschraube nach Anspruch 7, **dadurch gekennzeichnet, daß** das Halteelement (30; 74') ein elastisches Element ist.

9. Knochenschraube nach Anspruch 8, **dadurch gekennzeichnet, daß** Halteelement eine Schraubenfeder (30) oder ein Paket (74') von Tellerfedern (72') ist.

10. Knochenschraube nach einem der Ansprüche 7 bis 9, **dadurch gekennzeichnet, daß** das Halteelement (76") derart ausgebildet ist, daß eine äußere Abmessung und/oder ein Volumen des Halteelements durch Änderung mindestens einer Zustandsgröße in einer Umgebung der Knochenschraube (14) änderbar ist.

11. Knochenschraube nach Anspruch 10, **dadurch gekennzeichnet, daß** die mindestens eine Zustandsgröße eine Umgebungstemperatur, ein Umgebungsdruck, ein osmotischer Druck oder eine Feuchtigkeit der Umgebung ist.

12. Knochenschraube nach Anspruch 11, **dadurch gekennzeichnet, daß** das Halteelement (76") aus einem Memorymetall hergestellt ist.

13. Knochenschraube nach Anspruch 11, **dadurch gekennzeichnet, daß** das Halteelement (76") durch ein Fluid inflatierbar ist.

14. Knochenschraube nach Anspruch 11 oder 13, **dadurch gekennzeichnet, daß** das Halteelement (76") einen Innenraum aufweist, welcher über eine semipermeable Membran mit einer Umgebung der Knochenschraube (14) in Fluidverbindung steht.

15. Knochenschraube nach einem der voranstehenden Ansprüche, **dadurch gekennzeichnet, daß** das Sicherungselement (28) parallel zur Längsachse (23) verschiebbar am Schaft (22) gelagert ist.

16. Knochenschraube nach einem der voranstehenden Ansprüche, **dadurch gekennzeichnet, daß** die Sicherungselementaufnahme ein Sackloch (34) ist.

17. Knochenschraube nach einem der voranstehenden Ansprüche, **dadurch gekennzeichnet, daß** das Sicherungselement (28) drehformschlüssig in der Sicherungselementaufnahme (34) gehalten ist.

18. Knochenschraube nach einem der voranstehenden Ansprüche, **dadurch gekennzeichnet, daß** das Sicherungselement (28) einen Sicherungselementabschnitt (58) aufweist, welcher in Form eines Außenvielkant ausgebildet ist, und daß die Sicherungselementaufnahme (34) einen Sicherungselementaufnahmeabschnitt (40) aufweist, welcher in Form eines zum Außenvielkant korrespondierenden Innenvielkants ausgebildet ist.

19. Knochenschraube nach einem der Ansprüche 5 bis 18, **dadurch gekennzeichnet, daß** das erste Rastelement (44) an der Knochenschraube (14) in radialer Richtung bewegbar gelagert ist.

20. Knochenschraube nach einem der voranstehenden Ansprüche, **dadurch gekennzeichnet, daß** der Kopf (26) eine Mehrzahl quer zur Längsachse (23) bewegbarer Verriegelungselemente (48) umfaßt, welche in der Eingriffsstellung mit der Knochenplatte (12) in Eingriff stehen.

21. Knochenschraube nach Anspruch 20, **dadurch gekennzeichnet, daß** die Verriegelungselemente (48) das erste Rastelement tragen.

22. Knochenschraube nach einem der Ansprüche 20 oder 21, **dadurch gekennzeichnet, daß** der Kopf (26) lappenartige, durch sich parallel zur Längsachse (23) erstreckende Schlitze (46) getrennte Wandabschnitte (48) aufweist und daß die Wandabschnitte (48) die Verriegelungselemente bilden.

23. Knochenschraube nach einem der Ansprüche 20 bis 22, **dadurch gekennzeichnet, daß** die Verriegelungselemente (48) in der Grundstellung der Knochenschraube (14) die Eingriffsstellung einnehmen.

24. Knochenschraube nach einem der Ansprüche 20 bis 23, **dadurch gekennzeichnet, daß** zum Einführen des Sicherungselements (28) in die Sicherungselementaufnahme (34) die Verriegelungselemente (48) von der Eingriffsstellung radial nach außen bewegbar sind.

25. Knochenschraube nach einem der voranstehenden Ansprüche, **dadurch gekennzeichnet, daß** eine zweite Rastvorrichtung (52, 60) umfassend ein drittes und ein viertes, mit dem dritten Rastelement (60) zusammenwirkendes Rastelement (52) vorgesehen ist, daß das Sicherungselement (28) und der Kopf (26) jeweils ein Rastelement (52, 60) tragen und daß die zweite Rastvorrichtung (52, 60) eine Raststellung nach dem Einführen des Sicherungselements (28) in die Sicherungselementaufnahme (34) einnimmt.

26. Knochenschraube nach Anspruch 25, **dadurch gekennzeichnet, daß** das dritte Rastelement (60) eine in proximaler Richtung wirkende, radial nach außen abstehende Rastnase ist und daß das vierte Rastelement (52) eine radial nach innen vorstehende, in distaler Richtung wirkende Rastkante ist.

27. Knochenschraube nach Anspruch 25 oder 26, **dadurch gekennzeichnet, daß** die Verriegelungselemente (48) das vierte Rastelement (52) tragen.

28. Knochenschraube nach einem der Ansprüche 25 bis 27, **dadurch gekennzeichnet, daß** das dritte oder das vierte Rastelement (52, 60) den proximalen Anschlag bilden und in der Sicherungsstellung aneinander anliegen.

29. Knochenschraube nach einem der Ansprüche 25 bis 28, **dadurch gekennzeichnet, daß** sich an das dritte oder das vierte Rastelement (60) eine Aufgleitfläche (64) anschließt, an welcher das jeweils andere Rastelement (52) beim Einführen des Sicherungselements (28) in die Sicherungselementaufnahme (34) aufgleiten kann.

30. Knochenschraube nach einem der voranstehenden Ansprüche, **dadurch gekennzeichnet, daß** das Sicherungselement (28) in Form eines Kopfbolzens ausgebildet ist mit einem Bolzenschaft (58) und einem Bolzenkopf (56).

31. Knochenschraube nach Anspruch 30, **dadurch gekennzeichnet, daß** der Bolzenschaft (58) den Sicherungselementabschnitt bildet, welcher in Form eines Außenvielkant ausgebildet ist.

32. Knochenschraube nach einem der Ansprüche 30 oder 31, soweit diese direkt oder indirekt auf Anspruch 25 rückbezogen sind, **dadurch gekennzeichnet, daß** der Bolzenkopf (56) das dritte Rastelement (60) trägt.

33. Knochenschraube nach einem der voranstehenden Ansprüche, **dadurch gekennzeichnet, daß** die Knochenschraube (14) einen Werkzeugelementadapter (66) aufweist, welcher mit einem Einschraubwerkzeug (70) zum Einschrauben der Knochenschraube (14) in Eingriff bringbar ist.

34. Knochenschraube nach Anspruch 33, **dadurch gekennzeichnet, daß** das Sicherungselement (28) den Werkzeugadapter (66) trägt.

35. Knochenschraube nach einem der Ansprüche 33 oder 34, **dadurch gekennzeichnet, daß** der Werkzeugadapter (66) eine Ausnehmung ist, insbesondere in Form eines Schlitzes, eines Vielkants oder einer sternförmigen Vertiefung.

36. Knochenschraube nach einem der voranstehenden Ansprüche, **dadurch gekennzeichnet, daß** der Kopf (26) und der Schaft (22) einstückig ausgebildet sind.

37. Knochenschraube nach einem der voranstehenden Ansprüche, **dadurch gekennzeichnet, daß** der Schaft (22) mit einem Außengewinde (32) versehen ist, insbesondere mit einem selbstschneidenden Außengewinde.

38. Implantatsystem (10) umfassend mindestens eine Knochenplatte (12) mit mindestens einer Knochenschraubenaufnahme (18) und mindestens eine Knochenschraube (14), welche mit der Knochenschraubenaufnahme (18) in einer Eingriffsstellung in Eingriff bringbar ist zum lösbaren Verbinden der Knochenschraube (14) mit der Knochenplatte (12), **dadurch gekennzeichnet, daß** die Knochenschraube (14) eine Knochenschraube (14) nach einem der voranstehenden Ansprüche ist.

## Claims

1. Bone screw (14) with a shaft (22) defining a longitudinal axis (23) and a head (26) adapted to be brought into engagement with a bone screw receiving means (18) of a bone plate (12) for the releasable connection of the bone screw (14) to the bone plate (12), wherein a securing element (28) is provided for securing a connection between the bone screw (14) and the bone plate (12), wherein the bone screw (14) is adapted to be brought from a position of engagement, the bone screw (14) being held on the bone plate (12) in said position, into a release position, the bone screw (14) being releasable from the bone plate (12) in said position, wherein the securing element (28) is adapted to be brought from a non-securing position, the bone screw (14) being adapted to be brought into the release position in said non-securing position, into a securing position for securing the connection between the bone screw (14) and the bone plate (12), the bone screw (14) taking up the position of engagement in said securing position, the securing element (28) being supported on the bone screw (14) so as to be movable and the bone screw (14) takes up the position of engagement in a basic position, no external forces acting on the bone screw (14) in said basic position, wherein the shaft (22) has a securing element receiving means (34) and the securing element (28) is supported in the securing element receiving means (34), wherein following the insertion of the securing element (28) into the securing element receiving means (34) the securing element (28) is movable between a stop (42, 54) acting in a distal direction and a stop acting in a proximal direction and wherein the securing element (28) is held in the securing position in the basic position of the bone screw, **characterized in that** the securing element (28) is held in the securing position under tension.

2. Bone screw as defined in claim 1, **characterized in that** the bone screw (14) is designed so as to be rotationally symmetric or essentially rotationally symmetric in relation to the longitudinal axis (23).

3. Bone screw as defined in one of the preceding claims, **characterized in that** in the position of engagement the head (26) has a maximum external dimension transversely to the longitudinal axis (23) and in the release position an external dimension reduced in size in comparison with the maximum external dimension.

4. Bone screw as defined in claim 3, **characterized in that** in the position of engagement the head (26) has a maximum external diameter transversely to the longitudinal axis (23) and in the release position an external diameter reduced in size in comparison with the maximum external diameter.

5. Bone screw as defined in any one of the preceding claims, **characterized in that** the head (26) bears a first snap-in element (48) adapted to be interlocked with a second snap-in element (20) arranged on the bone plate (12) in the position of engagement.

6. Bone screw as defined in claim 5, **characterized in that** the first snap-in element (48) is an annular groove extending in circumferential direction and being open radially or an annular projection protruding radially outwards and extending in circumferential direction.

7. Bone screw as defined in any one of the preceding claims, **characterized in that** at least one holding element (30; 74'; 76") is provided for holding the securing element (28) in the securing position, said holding element being supported, on the one hand, on the shaft (22) and, on the other hand, on the securing element (28).

8. Bone screw as defined in claim 7, **characterized in that** the holding element (30; 74') is an elastic element.

9. Bone screw as defined in claim 8, **characterized in that** the holding element is a helical spring (30) or a stack (74') of plate springs (72').

10. Bone screw as defined in any one of claims 7 to 9, **characterized in that** the holding element (76") is designed in such a manner that an external dimension and/or a volume of the holding element is variable due to alteration to at least one state parameter in the surroundings of the bone screw (14).

11. Bone screw as defined in claim 10, **characterized in that** the at least one state parameter is ambient temperature, ambient pressure, osmotic pressure or humidity of the surroundings.

12. Bone screw as defined in claim 11, **characterized in that** the holding element (76") is produced from a memory metal.

13. Bone screw as defined in claim 11, **characterized in that** the holding element (76") is inflatable by means of a fluid.

14. Bone screw as defined in claim 11 or 13, **characterized in that** the holding element (76") has an interior space in fluid communication with the surroundings of the bone screw (14) via a semi-permeable membrane.

15. Bone screw as defined in any one of the preceding claims, **characterized in that** the securing element (28) is supported on the shaft (22) so as to be displaceable parallel to the longitudinal axis (23).

16. Bone screw as defined in any one of the preceding claims, **characterized in that** the securing element receiving means is a blind hole (34).

17. Bone screw as defined in any one of the preceding claims, **characterized in that** the securing element (28) is held in the securing element receiving means (34) in a non-rotational form-locking manner.

18. Bone screw as defined in any one of the preceding claims, **characterized in that** the securing element (28) has a securing element section (58) designed in the shape of an external polyhedron and that the securing element receiving means (34) has a securing element receiving means section (40) designed in the shape of an internal polyhedron corresponding to the external polyhedron.

19. Bone screw as defined in any one of claims 5 to 18, **characterized in that** the first snap-in element (44) is supported on the bone screw (14) so as to be movable in a radial direction.

20. Bone screw as defined in any one of the preceding claims, **characterized in that** the head (26) comprises a plurality of locking elements (48) movable transversely to the longitudinal axis (23), said locking elements being in engagement with the bone plate (12) in the position of engagement.

21. Bone screw as defined in claim 20, **characterized in that** the locking elements (48) bear the first snap-in element.

22. Bone screw as defined in one of claims 20 or 21, **characterized in that** the head (26) has tab-like wall sections (48) separated by slots (46) extending parallel to the longitudinal axis (23) and that the wall sections (48) form the locking elements.

23. Bone screw as defined in any one of claims 20 to 22, **characterized in that** the locking elements (48) take up the position of engagement in a basic position.

24. Bone screw as defined in any one of claims 20 to 23, **characterized in that** for the insertion of the securing element (28) into the securing element receiving means (34) the locking elements (48) are movable radially outwards from the position of engagement.

25. Bone screw as defined in any one of the preceding claims, **characterized in that** a second snap-in device (52, 60) comprising a third snap-in element and a fourth snap-in element (52) interacting with the third snap-in element (60) is provided, that the securing element (28) and the head (26) each bear a snap-in element (52, 60) and that the second snap-in device (52, 60) takes up a snap-in position after the insertion of the securing element (28) into the securing element receiving means (34).

26. Bone screw as defined in claim 25, **characterized in that** the third snap-in element (60) is a snap-in nose acting in a proximal direction and projecting radially outwards and that the fourth snap-in element (52) is a snap-in edge projecting radially inwards and acting in a distal direction.

27. Bone screw as defined in claim 25 or 26, **characterized in that** the locking elements (48) bear the fourth snap-in element (52).

28. Bone screw as defined in any one of claims 25 to 27, **characterized in that** the third or the fourth snap-in element (52, 60) forms the proximal stop and said elements abut on one another in the securing position.

29. Bone screw as defined in any one of claims 25 to 28, **characterized in that** a slide-on surface (64) adjoins the third or the fourth snap-in element (60), the respectively other snap-in element (52) being able to slide on said surface during the insertion of the securing element (28) into the securing element receiving means (34).

30. Bone screw as defined in any one of the preceding claims, **characterized in that** the securing element (28) is designed in the shape of a set bolt with a bolt shaft (58) and a bolt head (56).

31. Bone screw as defined in claim 30, **characterized in that** the bolt shaft (58) forms the securing element section designed in the shape of an external polyhedron.

32. Bone screw as defined in one of claims 30 or 31, as far as directly or indirectly dependent from claim 25, **characterized in that** the bolt head (56) bears the third snap-in element (60).

33. Bone screw as defined in any one of the preceding claims, **characterized in that** the bone screw (14) has a tool element adapter (66) adapted to be brought into engagement with a screw-in tool (70) for the screwing in of the bone screw (14).

34. Bone screw as defined in claim 33, **characterized in that** the securing element (28) bears the tool adapter (66).

35. Bone screw as defined in one of claims 33 or 34, **characterized in that** the tool adapter (66) is a recess, in particular, in the shape of a slot, a polyhedron or a star-shaped cavity.

36. Bone screw as defined in any one of the preceding claims, **characterized in that** the head (26) and the shaft (22) are designed in one piece.

37. Bone screw as defined in any one of the preceding claims, **characterized in that** the shaft (22) is provided with an external thread (32), in particular, with a self-cutting external thread.

38. Implant system (10) comprising at least one bone plate (12) with at least one bone screw receiving means (18) and at least one bone screw (14) adapted to be brought into engagement with the bone screw receiving means (18) in a position of engagement for the releasable connection of the bone screw (14) to the bone plate (12), **characterized in that** the bone screw (14) is a bone screw (14) as defined in any one of the preceding claims.

## Revendications

1. Vis à os (14) comprenant un corps allongé (22) définissant un axe longitudinal (23), et comprenant une tête (26), qui peut être amenée en prise avec un logement d'accueil de vis à os (18) d'une plaque à os (12) pour assurer une liaison amovible de la vis à os (14) avec la plaque à os (12), ensemble
dans lequel il est prévu un élément de blocage de sécurité (28) pour produire le blocage de sécurisation d'une liaison de la vis à os (14) et de la plaque à os (12),
dans lequel la vis à os (14) peut être amenée d'une position de prise, dans laquelle la vis à os (14) est maintenue sur la plaque à os (12), à une position de détachement dans laquelle la vis à os (14) peut être détachée de la plaque à os (12),
dans lequel l'élément de blocage de sécurité (28) peut être amené d'une position de déblocage dans laquelle la vis à os (14) peut être amenée dans la position de détachement, à une position de blocage de sécurité pour sécuriser la liaison entre la vis à os (14) et la plaque à os (12), dans laquelle la vis à os (14) prend la position de prise,
dans lequel l'élément de blocage de sécurité (28) est monté mobile sur la vis à os (14), et la vis à os (14) prend, dans une position de base dans laquelle aucune force extérieure n'agit sur elle, la position de prise, dans lequel le corps allongé (22) comporte un logement d'accueil d'élément de blocage de sécurité (34) et l'élément de blocage de sécurité (28) est monté dans le logement d'accueil d'élément de blocage de sécurité (34),
dans lequel, après l'introduction de l'élément de blocage de sécurité (28) dans le logement d'accueil d'élément de blocage de sécurité (34), l'élément de blocage de sécurité (28) est mobile dans la direction axiale, entre une butée (42, 54) agissant en direction distale et une butée agissant en direction proximale, et dans lequel l'élément de blocage de sécurité (28), dans la position de base de la vis à os (14), est maintenu dans la position de blocage de sécurité,
**caractérisée en ce que** dans la position de blocage de sécurité, l'élément de blocage de sécurité (28) est maintenu sous précontrainte.

2. Vis à os selon la revendication 1, **caractérisée en ce que** la vis à os (14) est d'une configuration à symétrie de rotation ou sensiblement à symétrie de rotation par rapport à l'axe longitudinal (23).

3. Vis à os selon l'une des revendications précédentes, **caractérisée en ce que** la tête (26) présente, dans la position de prise, une dimension extérieure maximale transversalement à l'axe longitudinal (23), et, dans la position de détachement, une dimension extérieure réduite par rapport à la dimension extérieure maximale.

4. Vis à os selon la revendication 3, **caractérisée en ce que** la tête (26) présente, dans la position de prise, un diamètre extérieur maximal transversalement à l'axe longitudinal (23), et dans la position de détachement, un diamètre extérieur réduit par rapport au diamètre extérieur maximal.

5. Vis à os selon l'une des revendications précédentes, **caractérisée en ce que** la tête (26) porte un premier élément d'encliquetage (48), qui dans la position de prise, peut venir s'encliqueter avec un deuxième élément d'encliquetage (20) agencé sur la plaque à os (12).

6. Vis à os selon la revendication 5, **caractérisée en ce que** le premier élément d'encliquetage (48) est une rainure annulaire ouverte radialement, qui s'étend dans la direction périphérique, ou bien une protubérance annulaire faisant saillie radialement vers l'extérieur et s'étendant dans la direction périphérique.

7. Vis à os selon l'une des revendications précédentes, **caractérisée en ce que** pour maintenir l'élément de blocage de sécurité (28) dans la position de blocage de sécurité, il est prévu au moins un élément de maintien (30; 74'; 76"), qui s'appuie, d'une part sur le corps allongé (22) et d'autre part sur l'élément de blocage de sécurité (28).

8. Vis à os selon la revendication 7, **caractérisée en ce que** l'élément de maintien (30; 74') est un élément élastique.

9. Vis à os selon la revendication 8, **caractérisée en ce que** l'élément de maintien est un ressort hélicoïdal (30) ou un empilement (74') de rondelles-ressort (72').

10. Vis à os selon l'une des revendications 7 à 9, **caractérisée en ce que** l'élément de maintien (76") est conçu de manière à ce qu'une dimension extérieure et/ou un volume de l'élément de maintien puisse varier sous l'effet de la variation d'au moins une grandeur d'état dans un environnement de la vis à os (14).

11. Vis à os selon la revendication 10, **caractérisée en ce que** ladite au moins une grandeur d'état est une température environnante, une pression environnante, une pression osmotique ou une humidité de l'environnement.

12. Vis à os selon la revendication 11, **caractérisée en ce que** l'élément de maintien (76") est fabriqué en un métal à mémoire.

13. Vis à os selon la revendication 11, **caractérisée en ce que** l'élément de maintien (76") peut être gonflé par un fluide.

14. Vis à os selon la revendication 11 ou la revendication 13, **caractérisée en ce que** l'élément de maintien (76") présente un espace intérieur, qui est en liaison fluidique avec un environnement de la vis à os (14), par l'intermédiaire d'une membrane semi-perméable.

15. Vis à os selon l'une des revendications précédentes, **caractérisée en ce que** l'élément de blocage de sécurité (28) est monté de manière à pouvoir coulisser sur le corps allongé (22), parallèlement à l'axe longitudinal (23).

16. Vis à os selon l'une des revendications précédentes, **caractérisée en ce que** le logement d'accueil d'élément de blocage de sécurité est un trou borgne (34).

17. Vis à os selon l'une des revendications précédentes, **caractérisée en ce que** l'élément de blocage de sécurité (28) est maintenu dans le logement d'accueil d'élément de blocage de sécurité (34) en y étant arrêté en rotation par complémentarité de formes.

18. Vis à os selon l'une des revendications précédentes, **caractérisée en ce que** l'élément de blocage de sécurité (28) comporte un tronçon d'élément de blocage de sécurité (58), qui est réalisé sous la forme d'un multi-pans extérieur, et **en ce que** le logement d'accueil d'élément de blocage de sécurité (34) présente un tronçon de logement d'accueil d'élément de blocage de sécurité (40), qui est réalisé sous la forme d'un multi-pans intérieur correspondant au multi-pans extérieur.

19. Vis à os selon l'une des revendications 5 à 18, **caractérisée en ce que** le premier élément d'encliquetage (44) est agencé sur la vis à os (14) de manière mobile dans la direction radiale.

20. Vis à os selon l'une des revendications précédentes, **caractérisée en ce que** la tête (26) comprend une pluralité d'éléments de verrouillage (48) mobiles transversalement à l'axe longitudinal (23), qui sont en prise avec la plaque à os (12) dans la position de prise.

21. Vis à os selon la revendication 20, **caractérisée en ce que** les éléments de verrouillage (48) portent le premier élément d'encliquetage.

22. Vis à os selon l'une des revendications 20 ou 21, **caractérisée en ce que** la tête (26) comporte des tronçons de paroi (48) en forme de languette, qui sont séparés par des fentes (46) s'étendant parallèlement à l'axe longitudinal (23), et **en ce que** les tronçons de paroi (48) forment les éléments de verrouillage.

23. Vis à os selon l'une des revendications 20 à 22, **caractérisée en ce que** les éléments de verrouillage (48) prennent la position de prise, dans la position de base de la vis à os (14).

24. Vis à os selon l'une des revendications 20 à 23, **caractérisée en ce que** pour introduire l'élément de blocage de sécurité (28) dans le logement d'accueil d'élément de blocage de sécurité (34), les éléments de verrouillage (48) sont mobiles radialement vers l'extérieur à partir de la position de prise.

25. Vis à os selon l'une des revendications précédentes, **caractérisée en ce qu'**il est prévu un deuxième dispositif d'encliquetage (52, 60) comprenant un troisième et un quatrième élément d'encliquetage (52), qui interagit avec le troisième élément d'encliquetage (60), et **en ce que** l'élément de blocage de sécurité (28) et la tête (26) portent respectivement un élément d'encliquetage (52, 60), et **en ce que** le deuxième dispositif d'encliquetage (52, 60) prend une position d'encliquetage après l'introduction de l'élément de blocage de sécurité (28) dans le logement d'accueil d'élément de blocage de sécurité (34).

26. Vis à os selon la revendication 25, **caractérisée en ce que** le troisième élément d'encliquetage (60) est un mentonnet d'encliquetage faisant saillie radialement vers l'extérieur et agissant en direction proximale, et **en ce que** le quatrième élément d'encliquetage (52) est un bord d'encliquetage en saillie radialement vers l'intérieur et agissant en direction distale.

27. Vis à os selon la revendication 25 ou la revendication 26, **caractérisée en ce que** les éléments de verrouillage (48) portent le quatrième élément d'encliquetage (52).

28. Vis à os selon l'une des revendications 25 à 27, **caractérisée en ce que** le troisième ou le quatrième élément d'encliquetage (52, 60) forment la butée proximale et sont en appui réciproque dans la position de blocage de sécurité.

29. Vis à os selon l'une des revendications 25 à 28, **caractérisée en ce qu'**au troisième ou au quatrième élément d'encliquetage (60) se raccorde une surface formant rampe de glissement (64), sur laquelle peut glisser respectivement l'autre élément d'encliquetage (52) lors de l'introduction de l'élément de blocage de sécurité (28) dans le logement d'accueil d'élément de blocage de sécurité (34).

30. Vis à os selon l'une des revendications précédentes, **caractérisée en ce que** l'élément de blocage de sécurité (28) est réalisé sous la forme d'un boulon à tête avec une tige de boulon (58) et une tête de boulon (56).

31. Vis à os selon la revendication 30, **caractérisée en ce que** la tige de boulon (58) forme le tronçon d'élément de blocage de sécurité, qui est réalisé sous la forme d'un multi-pans extérieur.

32. Vis à os selon l'une des revendications 30 ou 31, dans la mesure où celles-ci sont rattachées directement ou indirectement à la revendication 25, **caractérisée en ce que** la tête de boulon (56) porte le troisième élément d'encliquetage (60).

33. Vis à os selon l'une des revendications précédentes, **caractérisée en ce que** la vis à os (14) présente un adaptateur d'élément d'outil (66) pouvant être amené en prise avec un outil de vissage (70) pour visser la vis à os (14).

34. Vis à os selon la revendication 33, **caractérisée en ce que** l'élément de blocage de sécurité (28) porte l'adaptateur d'outil (66).

35. Vis à os selon l'une des revendications 33 ou 34, **caractérisée en ce que** l'adaptateur d'outil (66) est un évidement, notamment sous la forme d'une fente, d'un multi-pans ou d'un creux en forme d'étoile.

36. Vis à os selon l'une des revendications précédentes, **caractérisée en ce que** la tête (26) et le corps (22) sont réalisés d'un seul tenant.

37. Vis à os selon l'une des revendications précédentes, **caractérisée en ce que** le corps allongé (22) est muni d'un filetage extérieur (32), notamment d'un filetage extérieur auto-taraudant.

38. Système d'implant (10) comprenant au moins une plaque à os (12) avec au moins un logement d'accueil de vis à os (18), et au moins une vis à os (14), qui peut être amenée en prise avec le logement d'accueil de vis à os (18) dans une position de prise, en vue d'assurer une liaison amovible de la vis à os (14) avec la plaque à os (12), **caractérisé en ce que** la vis à os (14) est une vis à os (14) selon l'une des revendications précédentes.
